# EUROPÄISCHE PATENTSCHRIFT

(11) **EP 3 819 002 B1**
(45) Veröffentlichungstag und Bekanntmachung des Hinweises auf die Patenterteilung: **28.02.2024**
(21) Anmeldenummer: 20000399.4
(22) Anmeldetag: 04.11.2020
(51) Int. Cl.: A61M 16/12, A61M 16/00, A61M 16/20

(54) **BEATMUNGSGERÄT MIT EINER REGELUNGSEINHEIT ZUR REGELUNG EINER SAUERSTOFFKONZENTRATION IN EINEM ATEMGAS**
VENTILATOR INCLUDING A CONTROL UNIT FOR CONTROLLING OXYGEN CONCENTRATION IN A BREATHING GAS
APPAREIL RESPIRATOIRE COMPRENANT UNE UNITÉ DE RÉGULATION PERMETTANT DE RÉGULER UNE CONCENTRATION D'OXYGÈNE DANS UN GAZ RESPIRATOIRE

(30) Priorität: 11.11.2019 DE 102019130350
(43) Veröffentlichungstag der Anmeldung: 12.05.2021
(73) Patentinhaber: Löwenstein Medical Technology S.A., 2557 Luxembourg (LU)
(72) Erfinder: Kremeier, Peter, 76149 Karlsruhe (DE); Pulletz, Sven, 49090 Osnabrück (DE)
(74) Vertreter: Marx, Thomas

(56) Entgegenhaltungen:
- WO-A1-2017/079798
- US-A1- 2013 139 817
- US-A1- 2018 272 087

## Beschreibung

Die Erfindung betrifft ein Beatmungsgerät mit einer Regelungseinheit zur Regelung des Beatmungsgeräts nach Anspruch 1.

Beatmungsgeräte werden zur Beatmung von Lebewesen, insbesondere Menschen, eingesetzt. Üblicherweise unterstützen Beatmungsgeräte solche Lebewesen bei der Atmung, die selbst lediglich eine unzureichende oder aussetzende Eigenatmung aufweisen. Zur Unterstützung führt das Beatmungsgerät dem zu beatmenden Lebewesen ein Atemgas mit einem Beatmungsdruck zu. Die Zufuhr des Atemgases, d.h. die Beatmung, erfolgt in aller Regel periodisch mit einer Beatmungsfrequenz, die sich an einer üblichen Atemfrequenz oder einem Atemminutenvolumen des zu beatmenden Lebewesens orientieren kann. In Abhängigkeit der Atemfrequenz oder des Atemminutenvolumens ergibt sich auch ein mit dem Beatmungsgerät zu leistender Gasaustausch des zu beatmenden Lebewesens.

Zur Beatmung des Lebewesens mit dem Atemgas reichert das Beatmungsgerät eine Umgebungsluft mit einem Sauerstoffgas an. Es ist bekannt, mit dem Beatmungsgerät Atemgas mit einem geeigneten Feuchtegehalt und einer geeigneten Temperatur für die Beatmung bereitzustellen. Für solche Funktionen kann das Beatmungsgerät eine Befeuchtungseinheit und/oder eine Wärmeeinheit umfassen.

Eine unsachgemäße Beatmung der zu beatmenden Lebewesen kann zu körperlichen Schädigungen führen. So kann ein Sauerstoffmangel in dem Atemgas über mehrere Minuten zu irreversibler Schädigung der Gehirnzellen führen. Andererseits kann eine über mehrere Minuten andauernde überhöhte Sauerstoffkonzentration in dem Atemgas, insbesondere eine Sauerstoffkonzentration zwischen 98 % und 100 %, ebenfalls zu irreversiblen Schädigungen der Gehirnzellen führen, die Lunge dauerhaft schädigen oder die Sehkraft bis zur vollständigen Erblindung reduzieren. Mit einer Blutgasanalyse wird üblicherweise sichergestellt, dass das Beatmungsgerät bzw. die Regelung des Beatmungsgeräts korrekt eingestellt sind.

Hierzu wird dem zu beatmenden Lebewesen Blut entnommen und dessen Sauerstoff- und Kohlendioxidkonzentration bestimmt.

Für eine sichere und zuverlässige, d.h. physiologisch korrekte Beatmung mit dem Beatmungsgerät wird die Beatmung überwacht. Üblicherweise werden bei der Beatmung die Eigenschaften des Atemgases, der Gasaustausch und der Beatmungsdruck überwacht. Aus DE 10 2009 013 396 B3 ist eine Vorrichtung und ein Verfahren zur Steuerung einer Sauerstoffdosierung eines Beatmungsgerätes bekannt. Die DE 10 2009 013 396 B3 sieht vor, das Beatmungsgerät in Abhängigkeit eines Zeitverhaltens des Beatmungsgerätes, einer an dem Beatmungsgerät angeschlossenen Messanordnung zur Sauerstoffsättigungsmessung oder eines Patienten zu steuern. Weitere relevante Dokumente aus dem Stand der Technik sind WO 2017/079798 A1, US 2013/139817 A1 und US 2018/272087 A1.

Für eine weitergehende Verbesserung der sicheren Beatmung ist eine Verbesserung der Regelung eines Beatmungsgeräts erforderlich.

Der Erfindung liegt daher die Aufgabe zugrunde, eine verbesserte Regelung eines Beatmungsgeräts bereitzustellen, die eine gleichmäßigere Sauerstoffsättigung bei einem zu beatmenden Lebewesen und somit eine sicherere Beatmung sicherstellen kann.

Diese Aufgabe wird gemäß einem ersten Aspekt der Erfindung mit einem Verfahren zur Initialisierung einer Regelung eines Beatmungsgerätes nach Anspruch 1 gelöst.

Die Initialisierung der Regelung erfolgt, bevor mit einer Beatmung begonnen wird. Hierzu erfolgt das Einstellen der Sauerstoffgrenzkonzentration vorzugsweise durch den Anwender des Beatmungsgeräts. Dieser kann die Sauerstoffgrenzkonzentration beispielsweise über eine Benutzerschnittstelle des Beatmungsgeräts einstellen. Es ist denkbar, dass das Einstellen der Sauerstoffgrenzkonzentration ein Auswählen der Sauerstoffgrenzkonzentration aus einer hinterlegten Auswahlliste umfasst. Das Einstellen der Sauerstoffgrenzkonzentration kann insbesondere aber auch durch Eingabe eines Wertes der Sauerstoffgrenzkonzentration erfolgen.

Das Beatmungsgerät ist vorzugsweise derart ausgebildet, dass es während der Beatmung eine minimale alveoläre Ventilation garantieren kann. Eine minimale alveoläre Ventilation ist garantiert, wenn ein Atemzugvolumen von mindestens 4,4 ml/kg und/oder eine Atemfrequenz von mindestens 5 Atemzügen pro Minute möglich ist. Insoweit kommen insbesondere auch alle Beatmungsmodi in Frage, die die minimale alveoläre Ventilation garantieren. Insbesondere sind dies auch klassische druck- oder volumenkontrollierte Modi wie BILEVEL oder VCV, sowie Spontanmodi mit unterschiedlichen Arten der Druckunterstützung.

Die Initialisierung der Regelung hat den Vorteil, dass Fehlfunktionen oder eine unzureichende Signalqualität frühzeitigt erkannt werden. Dies verhindert eine Beatmung mit einer zu hohen oder zu niedrigen Sauerstoffkonzentration und verhindert (präventiv) Schädigungen des zu beatmenden Lebewesens.

In einer ersten bevorzugten Ausführungsform des Verfahrens liegt das einzustellende Sauerstoffsättigungszielintervall zwischen einer Sauerstoffsättigung des zu beatmenden Lebewesens von mindestens 85 % und maximal 99 %, wobei vorzugsweise einzustellende Sauerstoffsättigungszielintervalle im Bereich von 85 % - 88 %, 86 % - 89 %, 87 % - 90 %, 88 % - 91 %, 89 % - 92 %, 90 % - 93 %, 91 % - 94 %, 92 % - 95 %, 93% - 96 %, 94 % - 97 %, 95 % - 98 %, oder 96 % - 99 % liegen. Diese Sauerstoffsättigungszielintervalle sind insbesondere für die Beatmung geeignet. Insbesondere erlauben die Sauerstoffsättigungszielintervalle in vorteilhafterweise eine schnelle Auswahl und reduzieren Fehler durch die Eingabe der Werte durch den Anwender.

Gemäß einer weiteren bevorzugten Ausführungsform umfasst das Einstellen der Sauerstoffgrenzkonzentration in dem Atemgas den Schritt: Beaufschlagen der erfassten IST-Sauerstoffkonzentration mit einem Sauerstoffsicherheitsfaktor, wobei der Sauerstoffsicherheitsfaktor vorzugsweise 20 % entspricht. Weist beispielsweise ein Atemgas zur Beatmung eines Patienten eine erfasste IST-Sauerstoffkonzentration von 45 % auf, ergibt sich durch Beaufschlagung des Sauerstoffsicherheitsfaktors von 20 % eine einzustellende Sauerstoffgrenzkonzentration von 65 %. Überschreitet die IST-Sauerstoffkonzentration die eingestellte Sauerstoffgrenzkonzentration, wird vorzugsweise ein Alarm ausgelöst. Insbesondere läuft die Regelung im Übrigen weiter, kann aber von dem Anwender deaktiviert werden. Ferner kann es bevorzugt sein, dass die Regelung deaktiviert wird, wenn die Signalqualität der erfassten IST-Sauerstoffkonzentration und/oder der IST-Sauerstoffsättigung des zu beatmenden Lebewesens unzureichend ist.

Diese bevorzugte Ausführungsform hat den Vorteil, die Sauerstoffgrenzkonzentration standardisiert einzustellen. Insbesondere kann die Sauerstoffgrenzkonzentration durch die Regelung mit einem entsprechend hinterlegten Sauerstoffsicherheitsfaktor in vorteilhafterweise automatisiert berechnet und eingestellt werden.

In einer weiteren bevorzugten Fortbildung ist das Verfahren durch die Schritte gekennzeichnet: Starten der Beatmung durch Bestätigen der eingestellten Sauerstoffgrenzkonzentration in dem Atemgas, wenn die Signalqualität geeignet ist; und/oder Ausgeben einer Warninformation, wenn die Signalqualität ungeeignet ist. In vorteilhafter Weise erhält der Anwender unmittelbar eine Rückmeldung, ob das. Beatmungsgerät und die Regelung des Beatmungsgeräts für die sichere und korrekte Beatmung einsatzbereit sind. Diese bevorzugte Ausführungsform des Verfahrens informiert den Anwender über eine unzureichende Signalqualität. Dies hat den Vorteil, dass die Gefahr, falsch bzw. unzureichend zu beatmen, weiter reduziert wird.

Die Regelungseinheit des Beatmungsgerätes ist konfiguriert, die folgenden Schritte durchzuführen: Erfassen der IST-Sauerstoffsättigung des zu beatmenden Lebewesens für eine Beatmungszeit; Erfassen der IST-Sauerstoffkonzentration in dem Atemgas für eine Beatmungszeit; Festlegen eines Sauerstoffsättigungsziels; Vergleichen der IST-Sauerstoffsättigung mit dem Sauerstoffsättigungsziel; und Regeln der Sauerstoffkonzentration in dem Atemgas auf eine Sauerstoffzielkonzentration in Abhängigkeit des Vergleichs der IST-Sauerstoffsättigung mit dem Sauerstoffsättigungsziel, wobei das Regeln der Sauerstoffkonzentration in dem Atemgas umfasst: Regeln des Umgebungsluftstroms in der Luftleitung ; und/oder Regeln des Sauerstoffstroms in der Sauerstoffleitung; und/oder Regeln des Atemgases in der Atemgasleitung.

Insbesondere wird die die Sauerstoffkonzentration in dem Atemgas in einem Bereich von 21 % bis 100 % geregelt. Die Regelung kann in vorteilhafter Weise vorzugsweise unabhängig vom Beatmungsmodus eingesetzt werden. Die Regelung hat den Vorteil, dass die Beatmung automatisch erfolgt, insbesondere auf Grundlage der erfassten IST-Sauerstoffsättigung und erfassten IST-Sauerstoffkonzentration einerseits und dem festgelegten Sauerstoffsättigungsziel andererseits. Dies reduziert die manuelle Einflussnahme durch den Anwender und somit die Gefahr menschlicher Fehler in vorteilhafter Weise.

Die Regelung hat den Vorteil, dass Fehlfunktionen oder eine unzureichende Signalqualität frühzeitigt erkannt werden. Dies verhindert eine Beatmung mit einer zu hohen oder zu niedrigen Sauerstoffkonzentration und verhindert insoweit präventiv Schädigungen des zu beatmenden Lebewesens.

Gemäß einer weiteren bevorzugten Ausführungsform umfasst der Verfahrensschritt des Regelns der Sauerstoffkonzentration in dem Atemgas die Schritte: Erhöhen der Sauerstoffkonzentration, wenn mindestens seit einer ersten Wartezeit die Sauerstoffkonzentration des Atemgases nicht erhöht wurde, und Senken der Sauerstoffkonzentration, wenn mindestens seit einer zweiten Wartezeit die Sauerstoffkonzentration des Atemgases nicht abgesenkt wurde. Insbesondere ist die Regelung derart ausgebildet, dass sprunghafte Änderungen und/oder Artefakte im zeitlichen Verlauf der erfassten IST-Sauerstoffsättigung unberücksichtigt bleiben. Ferner bleiben im Vergleich zum üblichen bzw. natürlichen Verlauf einer Sauerstoffsättigung eines zu beatmenden Lebewesens insbesondere hochfrequente Änderungen im zeitlichen Verlauf der erfassten IST-Sauerstoffsättigung unberücksichtigt. Dies garantiert trotz einer unzureichenden Signalqualität der erfassten IST-Sauerstoffsättigung eine sichere Beatmung des zu beatmenden Lebewesens und verhindert sprunghafte und/oder schädigende Änderungen der tatsächlichen Sauerstoffsättigung des zu beatmenden Lebewesens.

Gemäß einer weiteren bevorzugten Ausführungsform ist die Regelung des Beatmungsgeräts gekennzeichnet durch den Schritt Prüfen der Signalqualität der erfassten IST-Sauerstoffsättigung.

Gemäß einer weiteren bevorzugten Fortbildung ist die Regelung des Beatmungsgeräts gekennzeichnet durch den Schritt Alarmieren. Der Verfahrensschritt des Alarmierens erfolgt, wenn: die erfasste IST-Sauerstoffsättigung unterhalb des festgelegten Sauerstoffsättigungsziels liegt; und/oder die erfasste IST-Sauerstoffsättigung oberhalb des festgelegten Sauerstoffsättigungsziels liegt; und/oder die geprüfte Signalqualität unzureichend ist, insbesondere, wenn nach einer Pufferzeit die Signalqualität nach erneutem Prüfen unzureichend ist. Die Signalqualität ist insbesondere unzureichend, wenn der zeitliche Verlauf der erfassten IST-Sauerstoffsättigung eine Vielzahl Artefakte und/oder sprunghafte Änderungen und/oder einen hochfrequenten zeitlichen Verlauf aufweist. Dieser Schritt des Alarmierens umfasst insbesondere den Schritt des Deaktivierens der Regelung, wenn die Sigrialqualität unzureichend ist. Diese bevorzugte Ausführungsform verhindert in vorteilhafter Weise eine Beatmung mit einer zu hohen oder zu niedrigen Sauerstoffkonzentration und verhindert Schädigungen des zu beatmenden Lebewesens.

In einer weiteren bevorzugten Fortbildung ist die Regelung gekennzeichnet durch die Schritte: Ausgeben an eine Benutzerschnittstelle des Beatmungsgeräts wenigstens einer folgenden Information: Verlauf der IST-Sauerstoffkonzentration in dem Atemgas; und/oder Dauer, während welcher die Sauerstoffkonzentration im Wesentlichen während der Regelung unverändert gehalten wurde; und/oder Dauer seit einer letzten Änderung der Sauerstoffkonzentration; und/oder die IST-Sauerstoffsättigung; und/oder das Sauerstoffsättigungsziel; und/oder die Sauerstoffgrenzkonzentration; und/oder durch den Schritt: Eingeben an der Benutzerschnittstelle des Beatmungsgeräts wenigstens eines folgenden Befehls: Pausieren der Regelung der Sauerstoffkonzentration in dem Atemgas, insbesondere Pausieren der Regelung der Sauerstoffkonzentration für eine Blutgasmessung; und/oder Ein- und/oder Ausschalten der Erfassung der IST-Sauerstoffsättigung. Die Eingabe eines genannten Befehls hat zur Folge, dass dieser Befehl durchgeführt wird, sprich dass beispielsweise die Regelung der Sauerstoffkonzentration in dem Atemgas pausiert wird. Vorzugsweise ist die Benutzerschnittstelle eine numerische Anzeige zum Ausgeben wenigstens einer der zuvor genannten Informationen und/oder zum Eingeben wenigstens einer der zuvor genannten Befehle. Diese bevorzugte Ausführungsform des Verfahrens hat insbesondere den Vorteil, dass ein Anwender, beispielsweise ein Arzt, Pfleger etc. schnell und einfach erkennen kann, ob bzw. wann es sinnvoll ist, eine Blutgasanalyse durchzuführen.

Nach einer weiteren bevorzugten Ausführungsform ist die Regelung dadurch gekennzeichnet, dass das Sauerstoffsättigungsziel innerhalb des eingestellten Sauerstoffsättigungszielintervalls liegt.

Das Festlegen des Sauerstoffsättigungsziels umfasst die folgenden Schritte: Bestimmen eines Mittelwertes des eingestellten Sauerstoffsättigungszielintervalls; und Festlegen des bestimmten Mittelwerts als Sauerstoffsättigungsziel.

Gemäß einer weiteren bevorzugten Fortbildung ist die Regelung dadurch gekennzeichnet, dass das Regeln der Sauerstoffkonzentration in dem Atemgas in Abhängigkeit des festgelegten Sauerstoffsättigungsziels des zu beatmenden Lebewesens mit einem PID-Regler erfolgt.

Gemäß einer weiteren bevorzugten Ausführungsform ist die Regelung dadurch gekennzeichnet, dass das Festlegen des Sauerstoffsättigungsziels die Schritte umfasst: Ermitteln eines gleitenden Mittelwertes der erfassten Sauerstoffsättigung über eine Messdauer; Festlegen des ermittelten gleitenden Mittelwertes der erfassten Sauerstoffsättigung als Sauerstoffsättigungsziel. Vorzugsweise beträgt die Messdauer 15 Sekunden. Die während der Messung für die Messdauer erfassten IST-Sauerstoffsättigungswerte bilden die Grundlage für den zu ermittelnden gleitenden Mittelwert.

Gemäß einer weiteren bevorzugten Ausführungsform ist die Regelung dadurch gekennzeichnet, dass das Erhöhen der Sauerstoffkonzentration die Schritte umfasst: Erhöhen der IST-Sauerstoffkonzentration auf 100 %, wenn die erfasste IST-Sauerstoffsättigung 10 % unterhalb des festgelegten Sauerstoffsättigungsziels liegt; und/oder Erhöhen der IST-Sauerstoffkonzentration um 15 %, wenn die erfasste IST-Sauerstoffsättigung 6 % - 10 % unterhalb des festgelegten Sauerstoffsättigungsziels liegt; und/oder Erhöhen der IST-Sauerstoffkonzentration um 10 %, wenn die erfasste IST-Sauerstoffsättigung 4 % - 6 % unterhalb des festgelegten Sauerstoffsättigungsziels liegt; und/oder Erhöhen der IST-Sauerstoffkonzentration um 5 %, wenn die erfasste IST-Sauerstoffsättigung 2 % - 3 % unterhalb des festgelegten Sauerstoffsättigungsziels liegt; und/oder Erhöhen der IST-Sauerstoffkonzentration um 1 %, wenn die erfasste IST-Sauerstoffsättigung 1 % - 2 % unterhalb des festgelegten Sauerstoffsättigungsziels liegt; und/oder das Senken der Sauerstoffkonzentration die Schritte umfasst: Senken der IST-Sauerstoffkonzentration auf 10 %, wenn die erfasste IST-Sauerstoffsättigung 10 % oder 6 % - 10 % oder 4 % - 6 % oberhalb des festgelegten Sauerstoffsättigungsziels liegt; und/oder Senken der IST-Sauerstoffkonzentration um 5 %, wenn die erfasste IST-Sauerstoffsättigung 2 % - 3 % oberhalb des festgelegten Sauerstoffsättigungsziels liegt; und/oder Senken der IST-Sauerstoffkonzentration um 1 %, wenn die erfasste IST-Sauerstoffsättigung 1 % - 2 % oberhalb des festgelegten Sauerstoffsättigungsziels liegt.

Beispielsweise wird bei einer erfassten IST-Sauerstoffsättigung von 78% und einem festgelegten Sauerstoffsättigungszielintervall von 88 % - 91 % die IST-Sauerstoffkonzentration in dem Atemgas auf 100 % erhöht. Auf Grundlage des beispielhaft angegebenen Sauerstoffsättigungszielintervalls wird ein Mittelwert von 89,5 % bestimmt, der als Sauerstoffsättigungsziel festgelegt wird. Für dieses Ausführungsbeispiel umfasst das Verfahren vorzugsweise den Schritt des Alarmierens.

Gemäß einer weiteren Ausführungsform der Regelung findet das Regeln der Sauerstoffkonzentration nur dann statt, wenn die Signalqualität der erfassten IST-Sauerstoffsättigung in Form eines Perfusionsindexes über einem bestimmten unteren Grenzwert liegt, wobei dieser untere Grenzwert zwischen 0,15% und 0,30% liegt, wobei das Regeln der Sauerstoffkonzentration in dem Atemgas die Schritte umfasst: Erhöhen der Sauerstoffkonzentration, wenn die erfasste IST-Sauerstoffsättigung unterhalb des festgelegten Sauerstoffsättigungsziels liegt, und/oder mindestens seit einer ersten Wartezeit die Sauerstoffkonzentratiön des Atemgases nicht erhöht wurde, und/oder ein Verlauf der erfassten IST-Sauerstoffsättigung innerhalb einer Trenddauer abnimmt; und/oder: Senken der Sauerstoffkonzentration, wenn: die erfasste IST-Sauerstoffsättigung oberhalb des festgelegten Sauerstoffsättigungsziels liegt, und/oder mindestens seit einer zweiten Wartezeit die Sauerstoffkonzentration des Atemgases nicht abgesenkt wurde, und/oder ein Verlauf der erfassten IST-Sauerstoffsättigung innerhalb einer Trenddauer zunimmt; und/oder: Halten der Sauerstoffkonzentration, wenn die erfasste IST-Sauerstoffsättigung im Wesentlichen dem festgelegten Sauerstoffsättigungsziel entspricht.

In manchen Ausführungsformen der Regelung findet eine Regelung der Sauerstoffkonzentration nur dann stattfindet, wenn die IST-Sauerstoffsättigung zwischen 50% und 100%, bevorzugt zwischen 85% und 98%, liegt.

Die Regelung ist in weiteren Fortbildungen dadurch gekennzeichnet, dass das Regeln der Sauerstoffkonzentration den Schritt umfasst, ein Zeitintervall abzuwarten, bevor der Schritt des Erhöhens oder Senkens der Sauerstoffkonzentration vorgenommen wird, wobei die ermittelten Werte der IST-Sauerstoffsättigung in dem Zeitintervall zum Regeln der Sauerstoffkonzentration nicht berücksichtigt werden und/oder in dem Zeitintervall der Schrott des Halten (203) ausgeführt wird und wobei das Zeitintervall zwischen 1 Sekunde und 20 Sekunden, bevorzugt zwischen 5 Sekunden und 10 Sekunden, beträgt.

Weitere Ausführungsformen der Regelung umfassen den Schritt des Überprüfens des Atemzugsvolumens umfasst, wobei der Schritt des Erhöhens und/oder der Schritt des Senkens des Regelns der Sauerstoffkonzentration erst erfolgt, wenn das Atemzugsvolumen als ausreichend groß bestimmt wird, wobei das Atemzugsvolumen durch eine Anpassung zumindest eines Beatmungsdruckes angepasst wird, wobei die Anpassung des Beatmungsdruckes durch ein Erhöhen des inspiratorischen Atemwegsdruckes (IPAP) und/oder Absenken des exspiratorischen Atemwegsdruckes (EPAP) erfolgt.

Insbesondere umfasst das Beatmungsgerät eine Benutzerschnittstelle zur Eingabe bzw. zum Aktivieren und Deaktivieren wenigstens einer folgenden Funktion: An- und Ausschalten der Erfassung der IST-Sauerstoffsättigung, Pausieren der Beatmung, Anzeigen des festgelegten Sauerstoffsättigungsziels, und/oder Anzeigen der Sauerstoffgrenzkonzentration. Ferner zeigt die bevorzugt umfasste Benutzerschnittstelle insbesondere wenigstens eine der folgenden auszugebenden Informationen: Verlauf der IST-Sauerstoffkonzentration in dem Atemgas; und/oder Dauer, während welcher die Sauerstoffkonzentration im Wesentlichen während der Regelung unverändert gehalten wurde; und/oder Dauer seit einer letzten Änderung der Sauerstoffkonzentration; und/oder die IST-Sauerstoffsättigung; und/oder das Sauerstoffsättigungsziel; und/oder die Sauerstoffgrenzkonzentration. Vorzugsweise ist die Benutzerschnittstelle eine numerische Anzeige.

Das erfindungsgemäße Beatmungsgerät umfasst in manchen Ausführungsformen eine Benutzerschnittstelle zum Ausgeben von Informationen und/oder zum Eingeben von Befehlen und/oder von Daten, wobei die Benutzerschnittstelle insbesondere eine numerische Anzeige.

Bevorzugte Ausführungsformen der Erfindung werden beispielhaft anhand der beiliegenden Figuren beschrieben. Es zeigen:
- Figur 1:: eine schematische Darstellung einer bevorzugten Ausführungsform eines Beatmungsgeräts;
- Figur 2:: eine schematische Darstellung einer Initialisierung einer Regelung eines Beatmungsgeräts; und
- Figur 3:: eine schematische Darstellung einer bevorzugten Ausführungsform einer Regelung eines Beatmungsgeräts.

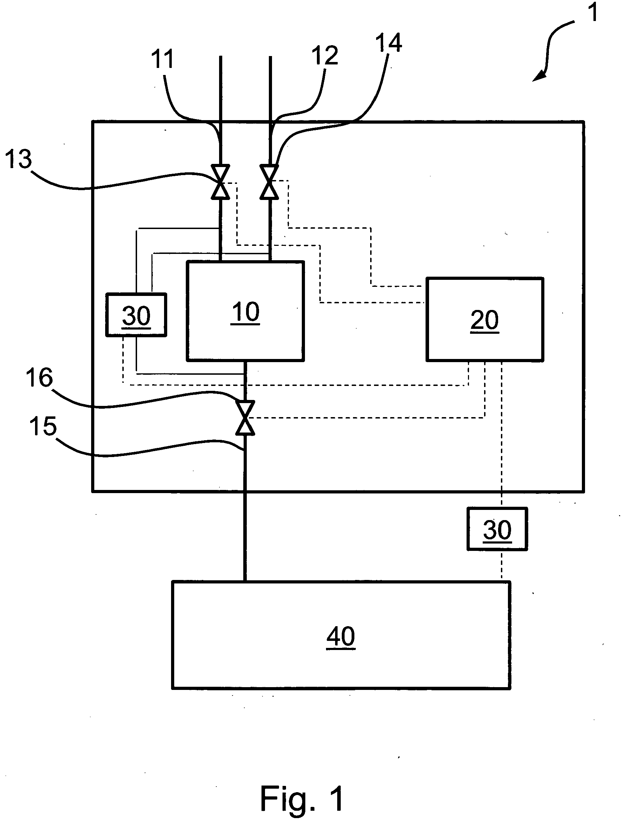

Figur 1 zeigt eine schematische Darstellung einer bevorzugten Ausführungsform eines Beatmungsgeräts 1. Das Beatmungsgerät 1 umfasst eine Mischeinheit 10. Die Mischeinheit 10 reichert eine Umgebungsluft mit Sauerstoff an, bis eine gewünschte Sauerstoffzielkonzentration erreicht ist. Diese, die Sauerstoffzielkonzentration aufweisende, mit Sauerstoff angereicherte Umgebungsluft stellt die Mischeinheit 10 als Atemgas zur Beatmung eines Lebewesens 40 zur Verfügung. In Krankenhäusern werden solche Beatmungsgeräte 1 beispielsweise zur Versorgung mit der Umgebungsluft und dem Sauerstoffgas an entsprechende Druckgasleitungen angeschlossen.

Die Mischeinheit 10 weist eine Luftleitung 11 auf. In der Luftleitung 11 kann die Umgebungsluft der Mischeinheit 10 während der Beatmung oder zur Initialisierung der Regelung zur Beatmung zugeführt werden. Ferner weist die Mischeinheit 10 eine Sauerstoffleitung 12 auf. Durch die Sauerstoffleitung 12 kann der Mischeinheit 10 Sauerstoff während der Beatmung oder zur Initialisierung der Regelung zur Beatmung zugeführt werden. Dem zu beatmenden Lebewesen 40 wird das die Sauerstoffzielkonzentration aufweisende Atemgas über eine Atemgasleitung 15 zur Beatmung zugeführt. Dafür ist die Atemgasleitung 15 mit der Mischeinheit 10 verbunden.

Zur Einstellung des Verhältnisses aus Umgebungsluft und Sauerstoffgas weist das Beatmungsgerät 1 in den jeweiligen Leitungen 11, 12 eine jeweilige Dosiereinheit auf. Mit der Luftdosiereinheit 13 lässt sich ein Luftstrom der zuzuführenden Umgebungsluft einstellen, also erhöhen, senken, oder halten. Mit der Sauerstoffdosiereinheit 14 kann ein Sauerstoffstrom der zuzuführenden Umgebungsluft eingestellt, also erhöht, gesenkt, oder gehalten werden. Ferner kann es bevorzugt sein, dass das Beatmungsgerät 1 in der Atemgasleitung 15 eine Atemgasdosiereinheit 16 zur Einstellung eines Atemgasstroms umfasst. Die jeweiligen Dosiereinheiten 13, 14, 16 sind vorzugsweise Stellventile.

Ferner umfasst das Beatmungsgerät 1 zur Einstellung der Sauerstoffzielkonzentration eine Messvorrichtung 30. Mit der Messvorrichtung 30 können Zustandsgrößen der der Mischeinheit 10 zuzuführenden Umgebungsluft in der Luftleitung 11, des der Mischeinheit 10 zuzuführende Sauerstoffgases in der Sauerstoffleitung 12 und des der Mischeinheit 10 abzuführenden Atemgases in der Atemgasleitung 14 erfasst werden. Hierzu kann die Messvorrichtung 30 beispielsweise einen oder mehrere Temperatur-, Druck-, und/oder Massestromsensoren aufweisen. Besonders bevorzugt lassen sich mit der Messvorrichtung 30 die jeweiligen Ströme in den jeweiligen Leitungen 11, 12, 15 erfassen und überwachen. Insbesondere ist die Messvorrichtung 30 ausgebildet, die Sauerstoffkonzentration des Atemgases in der Atemgasleitung 15 zu erfassen. Ferner ist die Messvorrichtung 30 ausgebildet, eine Sauerstoffsättigung des zu beatmenden Lebewesens 40 zu erfassen. Hierzu umfasst die Messvorrichtung 30 vorzugsweise ein Pulsoximeter, das an das zu beatmende Lebewesen 40 angeschlossen ist. Mithilfe der Messvorrichtung 30 und der jeweiligen Dosiereinheiten 13, 14, 16 wird die Umgebungsluft mit Sauerstoff angereichert, bis die Sauerstoffzielkonzentration in dem Atemgas erreicht ist.

Eine Regelungseinheit 20 ist mit der Messvorrichtung 30 signaltechnisch verbunden. Die Regelungseinheit 20 ist ferner mit den jeweiligen Dosiereinheiten 13, 14, 16 signaltechnisch verbunden. Mit Hilfe der Regelungseinheit 20 wird die Sauerstoffkonzentration in dem Atemgas in Abhängigkeit der erfassten IST-Sauerstoffsättigung geregelt. Beispielsweise kann ein Sauerstoffsättigungszielintervall bzw. ein Sauerstoffsättigungsziel im Bereich von 85 % bis 99 % liegen. Die Regelungseinheit 20 bzw. die auf der Regelungseinheit 20 laufende Regelung ist derart ausgebildet, dass auf kurze Ausreißer oder Artefakte der Erfassung der IST-Sauerstoffsättigung nicht oder zumindest nicht unmittelbar die Sauerstoffzielkonzentration geändert wird. Die Regelungseinheit 20 bzw. die auf der Regelungseinheit 20 laufende Regelung ist insbesondere derart ausgebildet, stark schwankende erfasste IST-Sauerstoffsättigungswerte oder einen zeitlichen Verlauf erfasster IST-Sauerstoffsättigungswerte mit vielen Artefakten für die Regelung der Sauerstoffzielkonzentration nicht zu berücksichtigen. Mit einem Pulsoximeter zur Erfassung der IST-Sauerstoffsättigungswerte lassen sich Artefakte im Vergleich zu anderen Messverfahren reduzieren und IST-Sauerstoffsättigungswerte mit einer hohen Signalqualität bereitstellen.

Insbesondere kann es bevorzugt sein, dass das Beatmungsgerät eine Benutzerschnittstelle (nicht dargestellt) aufweist. Dabei wird an die Benutzerschnittstelle des Beatmungsgeräts 1 wenigstens einer folgenden Information ausgegeben: Verlauf der IST-Sauerstoffkonzentration in dem Atemgas; und/oder Dauer, während welcher die Sauerstoffkonzentration im Wesentlichen während der Regelung unverändert gehalten wurde; und/oder Dauer seit einer letzten Änderung der Sauerstoffkonzentration; und/oder die IST-Sauerstoffsättigung; und/oder das Sauerstoffsättigungsziel; und/oder die Sauerstoffgrenzkonzentration. Zusätzlich oder alternativ erfolgt ein Eingeben an der Benutzerschnittstelle des Beatmungsgeräts 1 wenigstens eines folgenden Befehls: Pausieren der Regelung der Sauerstoffkonzentration in dem Atemgas, insbesondere Pausieren der Regelung der Sauerstoffkonzentration für eine Blutgasmessung; und/oder Ein- und/oder Ausschalten der Erfassung der IST-Sauerstoffsättigung. Die Benutzerschnittstelle erlaubt dem Anwender in besonders einfacher und schneller Weise relevante Informationen, wie etwa die zuvor beispielhaft, aber nicht abschließend genannten Informationen, zu entnehmen. Insbesondere befähigt eine solche Anzeige, beispielsweise betreffend die Ausgabe der Dauer seit der letzten Änderung der Sauerstoffkonzentration, den Anwender schnell und einfach zu erkennen, wann es sinnvoll ist, eine Blutgasanalyse durchzuführen.

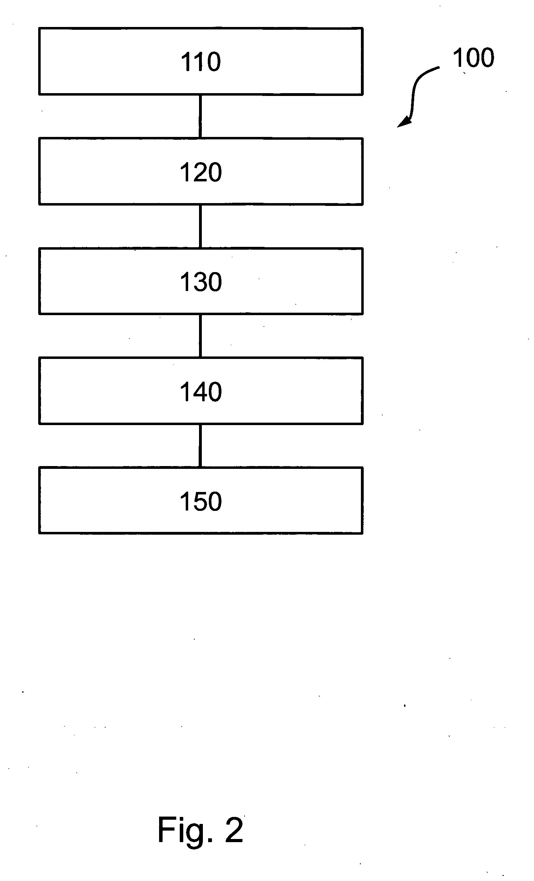

Figur 2 zeigt eine detailliertere Darstellung einer Initialisierung einer Regelung eines Beatmungsgeräts 1, wie beispielsweise in Figur 1 beschrieben. Das nicht beanspruchte Verfahren 100 umfasst die Schritte:
Einstellen 110 eines Sauerstoffsättigungszielintervalls eines zu beatmenden Lebewesens 40; Einstellen 120 einer Sauerstoffgrenzkonzentration in dem Atemgas; Erfassen 130 der IST-Sauerstoffsättigung des zu beatmenden Lebewesens 40 und Erfassen 140 der IST-Sauerstoffkonzentration in dem Atemgas für eine Initialisierungszeit; und Prüfen 150 der Signalqualität der erfassten IST-Sauerstoffsättigung.

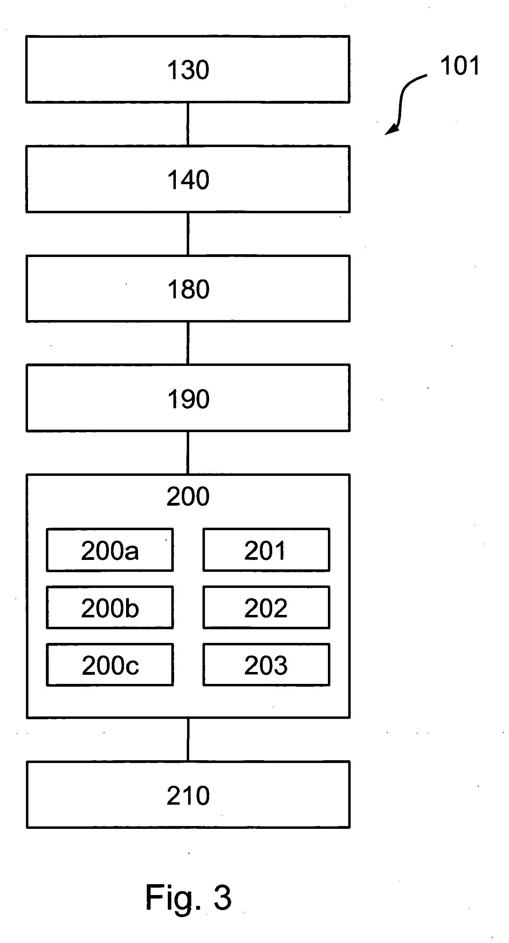

Figur 3 zeigt eine schematische Detaildarstellung einer bevorzugten Ausführungsform eines nicht beanspruchten Verfahrens 101 zur Regelung eines Beatmungsgeräts 1, wie beispielsweise in Figur 1 beschrieben. Das Verfahren 101 umfasst die Schritte: Erfassen 130 der IST-Sauerstoffsättigung des zu beatmenden Lebewesens 40 für eine Beatmungszeit; Erfassen 140 der IST-Sauerstoffkonzentration in dem Atemgas für eine Beatmungszeit; Festlegen 180 eines Sauerstoffsättigungsziels; Vergleichen 190 der IST-Sauerstoffsättigung mit dem Sauerstoffsättigungsziel; und Regeln 200 der Sauerstoffkonzentration in dem Atemgas auf eine Sauerstoffzielkonzentration in Abhängigkeit des Vergleichs der IST-Sauerstoffsättigung mit dem Sauerstoffsättigungsziel. Das Regeln der Sauerstoffkonzentration in dem Atemgas umfasst dabei: Regeln 200a des Umgebungsluftstroms in der Luftleitung 11; und/oder Regeln 200b des Sauerstoffstroms in der Sauerstoffleitung 12; und/oder Regeln 200c des Atemgases in der Atemgasleitung 15. Ferner umfasst das Regeln der Sauerstoffkonzentration das Erhöhen 201, Senken 202 und/oder Halten 203 der Sauerstoffkonzentration. Schließlich umfasst die in der Figur 3 gezeigte bevorzugte Ausführungsform des bevorzugten Verfahrens 101 zur Regelung des Beatmungsgeräts 1 den Verfahrensschritt des Alarmierens 210.

Je nachdem, ob die Sauerstoffkonzentration zu erhöhen, zu senken oder zu halten ist, werden der Sauerstoffstrom geregelt 200b und/oder der Luftstrom der Umgebungsluft geregelt 200a und/oder Atemgasstrom geregelt 200c.

Der Verfahrensschritt des Regelns 200 der Sauerstoffkonzentration umfasst insbesondere den Schritt des Erhöhens 201 der Sauerstoffkonzentration. Das Erhöhen 201 der Sauerstoffkonzentration erfolgt, wenn die erfasste IST-Sauerstoffsättigung unterhalb des festgelegten Sauerstoffsättigungsziels liegt und/oder mindestens seit einer ersten Wartezeit die Sauerstoffkonzentration des Atemgases nicht erhöht wurde und/oder ein Verlauf der erfassten IST-Sauerstoffsättigung innerhalb einer Trenddauer abnimmt. Der Verfahrensschritt des Regelns 200 der Sauerstoffkonzentration umfasst ferner insbesondere den Schritt des Senkens 202 der Sauerstoffkonzentration. Das Senken 202 der Sauerstoffkonzentration erfolgt, wenn die erfasste IST-Sauerstoffsättigung oberhalb des festgelegten Sauerstoffsättigungsziels liegt, und/oder wenn mindestens seit einer zweiten Wartezeit die Sauerstoffkonzentration des Atemgases nicht abgesenkt wurde, und/oder wenn ein Verlauf der erfassten IST-Sauerstoffsättigung innerhalb einer Trenddauer zunimmt. Ferner umfasst der Verfahrensschritt des Regelns 200 der Sauerstoffkonzentration insbesondere den Schritt des Haltens 203 der Sauerstoffkonzentration. Das Halten 203 der Sauerstoffkonzentration erfolgt, wenn die erfasste IST-Sauerstoffsättigung im Wesentlichen dem festgelegten Sauerstoffsättigungsziel entspricht.

Der Verfahrensschritt des Alarmierens 210 erfolgt insbesondere, wenn die erfasste IST-Sauerstoffsättigung unterhalb des festgelegten Sauerstoffsättigungsziels liegt; und/oder die erfasste IST-Sauerstoffsättigung oberhalb des festgelegten Sauerstoffsättigungsziels liegt; und/oder die geprüfte Signalqualität unzureichend ist, insbesondere, wenn nach einer Pufferzeit die Signalqualität nach erneutem Prüfen unzureichend ist.

Wenn die Signalqualität während des beispielsweise in Figur 3 beschriebenen Verfahrens 101 zur Regelung des Beatmungsgeräts unzureichend ist, wird vorzugsweise nach der Pufferzeit, beispielsweise nach 60 Sekunden, ein Alarm mittlerer Priorität ausgelöst. Insbesondere ist es denkbar, den Schritt des Alarmierens 210 zu eskalieren. Es kann beispielsweise bevorzugt sein, nach einer Pufferzeit von etwa 30 Sekunden eine Information als erste Eskalationsstufe bereitzustellen und nach einer Pufferzeit von etwa 90 Sekunden, wie zuvor beschrieben, den Alarm mittlerer Priorität auszulösen.

Die Signalqualität ist insbesondere unzureichend, wenn der zeitliche Verlauf der zu erfassenden IST-Sauerstoffsättigung stark bzw. hochfrequent schwankt und/oder Artefakte aufweist, die nicht dem üblichen bzw. natürlichen zeitlichen Verlauf der Sauerstoffsättigung eines zu beatmenden Lebewesens 40 entsprechen.

Die Signalqualität kann beispielsweise über den Perfusionsindex bestimmt werden, welcher das Verhältnis des pulsierenden Anteils und des nicht-pulsierenden Anteils des Infrarotsignals des Pulsoximeters wiedergibt. Das Regeln (200) der Sauerstoffkonzentration findet beispielsweise nur dann statt, wenn die Signalqualität in einem ausreichend guten Bereich liegt. In manchen Ausführungsformen muss beispielsweise auch die IST-Sauerstoffsättigung innerhalb vorgegebener Grenzwerte liegen, damit ein Regeln (200) stattfindet. Eine ausreichend gute Signalqualität in Form des Perfusionsindexes liegt beispielsweise vor, wenn der Perfusionsindex über 0,2% liegt, in manchen Ausführungsformen kann auch 0,25% als untere Grenze für eine gute Signalqualität angesehen werden. Eine ausreichende Signalqualität liegt insbesondere dann nicht mehr vor, wenn der Perfusionsindex unter 0,15% liegt. Die Grenzwerte der IST-Sauerstoffsättigung können beispielsweise zwischen 50% und 100% liegen, bevorzugt zwischen 85% und 98%.

In manchen Ausführungsformen erfolgt das Regeln (200) der Sauerstoffkonzentration erweiternd oder alternativ nur dann, wenn eine maximale Anzahl von Artefakten, wie zum Beispiel Bewegungsartefakten, in einem Zeitintervall unterschritten wird. Zum Beispiel können Artefakte auch automatisch nicht berücksichtigt werden, sodass zu Zeiten die erkannten Artefakte keine Berücksichtigung der gemessenen IST-Sauerstoffsättigung stattfindet.

Beispielsweise erfolgt das Regeln (200) der Sauerstoffkonzentration erst, nachdem ein gewisses Zeitintervall nach dem letzten Regeln (200) der Sauerstoffkonzentration abgelaufen ist und IST-Sauerstoffsättigungswerte aufgenommen wurden. In manchen Ausführungsformen findet nach einer Veränderung der Sauerstoffkonzentration (Erhöhen (201) und/oder Senken (202)) alternativ der Schritt des Haltens (203) der Sauerstoffkonzentration für das Zeitintervall statt.

Die ermittelten Werte der IST-Sauerstoffsättigung innerhalb des Zeitintervalls werden dabei nicht zum Regeln (200) der Sauerstoffkonzentration berücksichtigt. Alternativ kann in dem Zeitintervall nach dem Schritt des Erhöhens (201) und/oder Senkens (202) grundsätzlich der Schritt des Haltens (203) für das Zeitintervall folgen. Dieses Zeitintervall wird vorgegeben um den Effekt der Regelung der Sauerstoffkonzentration erkennen zu können. Beispielsweise beträgt das Zeitintervall zwischen 1 Sekunde und 20 Sekunden, zum Beispiel zwischen 5 Sekunden und 10 Sekunden. Das Zeitintervall, welches abgewartet (und/oder der Schritt des Haltens (203) durchgeführt wird) wird bis ein erneutes Regeln (200) in Form von Erhöhen (201) oder Senken (202) der Sauerstoffkonzentration durchgeführt wird, hängt beispielsweise auch von der zuvor ermittelten Änderung der IST-Sauerstoffsättigung ab. Wird beispielsweise ein Abfall der IST-Sauerstoffsättigung registriert, so kann das Zeitintervall kürzer ausfallen als wenn keine oder nur eine geringfügige Veränderung, beispielsweise von 3% oder weniger, ermittelt wird.

Das Regeln (200) beziehungsweise die Schritte des Erhöhens (201) und/oder des Senkens (202) erfolgt in manchen Ausführungsformen beispielsweise nach der Überprüfung des Atemzugvolumens. Ist das Atemzugsvolumen zu klein, kann dies beispielsweise eine Ursache für eine geringe IST-Sauerstoffsättigung sein, selbst wenn die Sauerstoffkonzentration hoch ist. Ist beispielsweise keine Zunahme der IST-Sauerstoffsättigung zu verzeichnen, nachdem die Sauerstoffkonzentration erhöht wurde, so kann ein Überprüfen des Atemzugsvolumens durchgeführt werden. Ist das Atemzugsvolumen zu klein, also nicht ausreichend groß, wird über die Anpassung der Beatmungsdrücke, zum Beispiel dem exspiratorischen Atemwegsdruck (EPAP) und dem inspiratorischen Atemwegsdruck (IPAP), das Atemzugsvolumen erhöht. Vorzugweise wird insbesondere der IPAP erhöht, in manchen Ausführungsformen wird gleichzeitig der EPAP in einem geringen Maße verringert. Zum Beispiel kann der EPAP auch konstant gehalten werden, während der IPAP erhöht wird. Nach der Erhöhung der Atemwegsdrücke wird gegebenenfalls die Sauerstoffkonzentration weiter erhöht um das Sauerstoffsättigungszielintervall zu erreichen. Das zu erreichende Atemzugsvolumen hängt dabei beispielsweise stark von dem zu beatmenden Lebewesen ab, zum Beispiel ist das Atemzugsvolumen für präadulte Lebewesen geringer als das für adulte Lebewesen.

### BEZUGSZEICHEN

- 1: Beatmungsgerät
- 10: Mischeinheit
- 11: Luftleitung
- 12: Sauerstoffleitung
- 13: Luftdosiereinheit
- 14: Sauerstoffdosiereinheit
- 15: Atemgasleitung
- 16: Atemgasdosiereinheit
- 20: Regelungseinheit
- 30: Messvorrichtung
- 40: zu beatmendes Lebewesen; Mensch
- 100: Verfahren zur Initialisierung einer Regelung eines Beatmungsgeräts
- 101: Verfahren zur Regelung eines Beatmungsgeräts
- 110: Einstellen eines Sauerstoffsättigungszielintervalls
- 120: Einstellen einer Sauerstoffgrenzkonzentration
- 130: Erfassen einer IST-Sauerstoffsättigung
- 140: Erfassen einer IST-Sauerstoffkonzentration
- 150: Prüfen einer Signalqualität
- 180: Festlegen eines Sauerstoffsättigungsziels
- 190: Vergleichen der IST-Sauerstoffsättigung mit dem Sauerstoffsättigungsziel
- 200: Regeln der Sauerstoffkonzentration auf eine Sauerstoffzielkonzentration
- 200a: Regeln des Umgebungsluftstroms in der Luftleitung
- 200b: Regeln des Sauerstoffstroms in der Sauerstoffleitung; und/oder
- 200c: Regeln des Atemgases in der Atemgasleitung
- 201: Erhöhen der Sauerstoffkonzentration
- 202: Senken der Sauerstoffkonzentration
- 203: Halten der Sauerstoffkonzentration
- 210: Alarmieren

## Patentansprüche

1. Beatmungsgerät (1) zur Beatmung eines Lebewesens (40), umfassend
- eine Mischeinheit (10) zur Anreicherung einer Umgebungsluft mit einem Sauerstoffgas auf eine Sauerstoffzielkonzentration und zur Bereitstellung der auf die Sauerstoffzielkonzentration angereicherten Umgebungsluft als Atemgas;
- eine Luftleitung (11), die mit der Mischeinheit (10) verbunden und ausgebildet ist, die Umgebungsluft der Mischeinheit (10) zuzuführen, wobei das Beatmungsgerät (1) eine Luftdosiereinheit (13) zur Einstellung des Luftstroms umfasst;
- eine Sauerstoffleitung (12), die mit der Mischeinheit (10) verbunden und ausgebildet ist, das Sauerstoffgas der Mischeinheit (10) zuzuführen, wobei das Beatmungsgerät (1) eine Sauerstoffdosiereinheit (14) zur Einstellung des Sauerstoffgasstroms umfasst;
- eine Atemgasleitung (15), die mit der Mischeinheit (10) verbunden ist und ausgebildet ist, das Atemgas aus der Mischeinheit (10) abzuführen, wobei das Beatmungsgerät (1) vorzugsweise eine Atemgasdosiereinheit (16) zur Einstellung eines Atemgasstroms umfasst;
- eine Regelungseinheit (20) zum Steuern des Beatmungsgeräts (1) und zur Einstellung der Sauerstoffzielkonzentration in dem Atemgas in Abhängigkeit von der IST-Sauerstoffsättigung und/oder dem Sauerstoffsättigungsziel;
- eine Messvorrichtung (30) zur Erfassung der IST-Sauerstoffsättigung des zu beatmenden Lebewesens (40) und/oder der IST-Sauerstoffkonzentration, wobei die Messvorrichtung (30) zur Erfassung der IST-Sauerstoffsättigung vorzugsweise ein Pulsoximeter umfasst; und
- eine Benutzerschnittstelle zum Ausgeben von Informationen und/oder Eingeben von Befehlen und/oder Daten, wobei die Benutzerschnittstelle insbesondere eine numerische Anzeige umfasst;
wobei die Regelungseinheit (20) konfiguriert ist, um die folgenden Schritte durchzuführen:
- Erfassen (130) der IST-Sauerstoffsättigung des zu beatmenden Lebewesens (40) für eine Beatmungszeit;
- Erfassen (140) der IST-Sauerstoffkonzentration in dem Atemgas für eine Beatmungszeit;
- Festlegen (180) eines Sauerstoffsättigungsziels;
- Vergleichen (190) der IST-Sauerstoffsättigung mit dem Sauerstoffsättigungsziel; und
- Regeln (200) der Sauerstoffkonzentration in dem Atemgas auf eine Sauerstoffzielkonzentration in Abhängigkeit vom Vergleich der IST-Sauerstoffsättigung mit dem Sauerstoffsättigungsziel, sodass die Beatmung auf Grundlage der erfassten IST-Sauerstoffsättigung und der erfassten IST-Sauerstoffkonzentration einerseits und des festgelegten Sauerstoffsättigungsziels andererseits automatisch erfolgt, wobei das Regeln der Sauerstoffkonzentration in dem Atemgas umfasst:
- Regeln (200a) des Umgebungsluftstroms in der Luftleitung (11); und/oder
- Regeln (200b) des Sauerstoffstroms in der Sauerstoffleitung (12); und/oder
- Regeln (200c) des Atemgases in der Atemgasleitung (15);
- Prüfen (150) der Signalqualität der erfassten IST-Sauerstoffsättigung;
**dadurch gekennzeichnet,**
**dass** das Regeln (200) der Sauerstoffkonzentration in dem Atemgas ferner umfasst:
- Erhöhen (201) der Sauerstoffkonzentration, wenn mindestens seit einer ersten Wartezeit die Sauerstoffkonzentration des Atemgases nicht erhöht wurde; und/oder
- Senken (202) der Sauerstoffkonzentration, wenn mindestens seit einer zweiten Wartezeit die Sauerstoffkonzentration des Atemgases nicht abgesenkt wurde;
und **dass** das Festlegen (108) des Sauerstoffsättigungsziels umfasst:
- Bestimmen eines Mittelwertes eines eingestellten Sauerstoffsättigungszielintervalls; und
- Festlegen des bestimmten Mittelwerts als Sauerstoffsättigungsziel.

2. Beatmungsgerät (1) nach Anspruch 1, wobei die Regelungseinheit (20) konfiguriert ist, um den folgenden Schritt durchzuführen:
- Alarmieren (210), wenn
- die erfasste IST-Sauerstoffsättigung unterhalb des festgelegten Sauerstoffsättigungsziels liegt und/oder
- die erfasste IST-Sauerstoffsättigung oberhalb des festgelegten Sauerstoffsättigungsziels liegt und/oder
- die geprüfte Signalqualität unzureichend ist, insbesondere wenn nach einer Pufferzeit die Signalqualität nach erneutem Prüfen unzureichend ist;
und/oder den folgenden Schritt durchzuführen:
- Ausgeben wenigstens einer der folgenden Informationen an eine Benutzerschnittstelle des Beatmungsgeräts (1):
- Verlauf der IST-Sauerstoffkonzentration in dem Atemgas,
- Dauer, während welcher die Sauerstoffkonzentration im Wesentlichen während der Regelung unverändert gehalten wurde,
- Dauer seit einer letzten Änderung der Sauerstoffkonzentration,
- die IST-Sauerstoffsättigung,
- das Sauerstoffsättigungsziel,
- die Sauerstoffgrenzkonzentration;
und/oder den folgenden Schritt durchzuführen:
- Eingeben wenigstens eines der folgenden Befehle an der Benutzerschnittstelle des Beatmungsgeräts (1):
- Pausieren der Regelung der Sauerstoffkonzentration in dem Atemgas, insbesondere Pausieren der Regelung der Sauerstoffkonzentration für eine Blutgasmessung;
- Ein- und/oder Ausschalten der Erfassung der IST-Sauerstoffsättigung.

3. Beatmungsgerät (1) nach einem der vorhergehenden Ansprüche, wobei das Erhöhen (201) der Sauerstoffkonzentration wenigstens einen der folgenden Schritte umfasst:
- Erhöhen der IST-Sauerstoffkonzentration auf 100 %, wenn die erfasste IST-Sauerstoffsättigung 10 % unterhalb des festgelegten Sauerstoffsättigungsziels liegt;
- Erhöhen der IST-Sauerstoffkonzentration um 15 %, wenn die erfasste IST-Sauerstoffsättigung 6 % bis 10 % unterhalb des festgelegten Sauerstoffsättigungsziels liegt;
- Erhöhen der IST-Sauerstoffkonzentration um 10 %, wenn die erfasste IST-Sauerstoffsättigung 4 % bis 6 % unterhalb des festgelegten Sauerstoffsättigungsziels liegt;
- Erhöhen der IST-Sauerstoffkonzentration um 5 %, wenn die erfasste IST-Sauerstoffsättigung 2 % bis 3 % unterhalb des festgelegten Sauerstoffsättigungsziels liegt;
- Erhöhen der IST-Sauerstoffkonzentration um 1 %, wenn die erfasste IST-Sauerstoffsättigung 1 % bis 2 % unterhalb des festgelegten Sauerstoffsättigungsziels liegt;
und/oder wobei das Senken (202) der Sauerstoffkonzentration wenigstens einen der folgenden Schritte umfasst:
- Senken der IST-Sauerstoffkonzentration auf 10 %, wenn die erfasste IST-Sauerstoffsättigung 10 % oder 6 % bis 10 % oder 4 % bis 6 % oberhalb des festgelegten Sauerstoffsättigungsziels liegt;
- Senken der IST-Sauerstoffkonzentration um 5 %, wenn die erfasste IST-Sauerstoffsättigung 2 % bis 3 % oberhalb des festgelegten Sauerstoffsättigungsziels liegt;
- Senken der IST-Sauerstoffkonzentration um 1 %, wenn die erfasste IST-Sauerstoffsättigung 1 % bis 2 % oberhalb des festgelegten Sauerstoffsättigungsziels liegt.

4. Beatmungsgerät (1) nach einem der vorhergehenden Ansprüche, wobei das Erhöhen (201) der Sauerstoffkonzentration erfolgt, wenn ferner
- die erfasste IST-Sauerstoffsättigung unterhalb des festgelegten Sauerstoffsättigungsziels liegt und/oder
- ein Verlauf der erfassten IST-Sauerstoffsättigung innerhalb einer Trenddauer abnimmt;
und/oder wobei das Senken (202) der Sauerstoffkonzentration erfolgt, wenn ferner
- die erfasste IST-Sauerstoffsättigung oberhalb des festgelegten Sauerstoffsättigungsziels liegt und/oder
- ein Verlauf der erfassten IST-Sauerstoffsättigung innerhalb einer Trenddauer zunimmt;
und/oder wobei das Regeln (200) der Sauerstoffkonzentration in dem Atemgas ferner umfasst: Halten (203) der Sauerstoffkonzentration, wenn die erfasste IST-Sauerstoffsättigung im Wesentlichen dem festgelegten Sauerstoffsättigungsziel entspricht.

5. Beatmungsgerät (1) nach Anspruch 4, wobei das Regeln (200) der Sauerstoffkonzentration nur dann stattfindet, wenn die Signalqualität der erfassten IST-Sauerstoffsättigung in Form eines Perfusionsindexes über einem bestimmten unteren Grenzwert liegt, wobei dieser untere Grenzwert zwischen 0,15 % und 0,30 % liegt.

6. Beatmungsgerät (1) nach einem der vorhergehenden Ansprüche, wobei das Regeln (200) der Sauerstoffkonzentration den Schritt umfasst, ein Zeitintervall abzuwarten, bevor der Schritt des Erhöhens (201) oder Senkens (202) der Sauerstoffkonzentration vorgenommen wird, wobei die ermittelten Werte der IST-Sauerstoffsättigung in dem Zeitintervall zum Regeln der Sauerstoffkonzentration nicht berücksichtigt werden und/oder in dem Zeitintervall ein Schritt des Haltens (203) der Sauerstoffkonzentration ausgeführt wird, wenn die erfasste IST-Sauerstoffsättigung im Wesentlichen dem festgelegten Sauerstoffsättigungsziel entspricht, und wobei das Zeitintervall zwischen 1 Sekunde und 20 Sekunden, bevorzugt zwischen 5 Sekunden und 10 Sekunden, beträgt.

7. Beatmungsgerät (1) nach einem der vorhergehenden Ansprüche, wobei das Verfahren (101) einen Schritt des Überprüfens des Atemzugsvolumens umfasst, wobei der Schritt des Erhöhens (201) und/oder der Schritt des Senkens (202) des Regelns (200) der Sauerstoffkonzentration erst erfolgt, wenn das Atemzugsvolumen als ausreichend groß bestimmt wird, wobei das Atemzugsvolumen durch eine Anpassung zumindest eines Beatmungsdruckes angepasst wird, wobei die Anpassung des Beatmungsdruckes durch ein Erhöhen des inspiratorischen Atemwegsdruckes (IPAP) und/oder Absenken des exspiratorischen Atemwegsdruckes (EPAP) erfolgt.

8. Beatmungsgerät (1) nach Anspruch 1, wobei die Regelungseinheit (20) konfiguriert ist, um die folgenden Schritte eines Verfahrens (100) zur Initialisierung der Regelung des Beatmungsgeräts (1) durchzuführen:
- Einstellen (110) eines Sauerstoffsättigungszielintervalls eines zu beatmenden Lebewesens (40);
- Einstellen (120) einer Sauerstoffgrenzkonzentration in dem Atemgas;
- Erfassen (130) der IST-Sauerstoffsättigung des zu beatmenden Lebewesens (40) und/oder Erfassen (140) der IST-Sauerstoffkonzentration in dem Atemgas für eine Initialisierungszeit; und
- Prüfen (150) einer Signalqualität der erfassten IST-Sauerstoffsättigung; wobei das Sauerstoffsättigungsziel innerhalb des eingestellten Sauerstoffsättigungszielintervalls liegt.

9. Beatmungsgerät (1) nach Anspruch 8, wobei das einzustellende Sauerstoffsättigungszielintervall zwischen einer Sauerstoffsättigung des zu beatmenden Lebewesens (40) von mindestens 85 % und maximal 99 % liegt, wobei vorzugsweise einzustellende Sauerstoffsättigungszielintervalle im Bereich von 85 % bis 88 %, 86 % bis 89 %, 87 % bis 90 %, 88 % bis 91 %, 89 % bis 92 %, 90 % bis 93 %, 91 % bis 94 %, 92 % bis 95 %, 93 % bis 96 %, 94 % bis 97 %, 95 % bis 98 % oder 96 % bis 99 % liegen.

10. Beatmungsgerät (1) nach Anspruch 8 oder 9, wobei das Einstellen (120) der Sauerstoffgrenzkonzentration in dem Atemgas umfasst:
- Beaufschlagen der erfassten IST-Sauerstoffkonzentration mit einem Sauerstoffsicherheitsfaktor, wobei der Sauerstoffsicherheitsfaktor vorzugsweise 20 % entspricht.

11. Beatmungsgerät (1) nach einem der Ansprüche 8 bis 10, wobei die Regelungseinheit (20) konfiguriert ist, um wenigstens einen der folgenden Schritte durchzuführen:
- Starten der Beatmung durch Bestätigen der eingestellten Sauerstoffgrenzkonzentration in dem Atemgas, wenn die Signalqualität geeignet ist;
- Ausgeben einer Warninformation, wenn die Signalqualität ungeeignet ist.

## Claims

1. A ventilator (1) for ventilating a living being (40), comprising
- a mixing unit (10) for enriching ambient air with an oxygen gas to a target oxygen concentration and for providing the ambient air enriched to the target oxygen concentration as the breathing gas;
- an air line (11) which is connected to the mixing unit (10) and is designed to supply the ambient air to the mixing unit (10), wherein the ventilator (1) comprises an air dosing unit (13) for setting the air flow;
- an oxygen line (12) which is connected to the mixing unit (10) and is designed to supply the oxygen gas to the mixing unit (10), wherein the ventilator (1) comprises an oxygen dosing unit (14) for setting the oxygen gas flow;
- a breathing gas line (15) which is connected to the mixing unit (10) and is designed to draw the breathing gas off from the mixing unit (10), wherein the ventilator (1) preferably comprises a breathing gas dosing unit (16) for setting a breathing gas flow;
- a regulation unit (20) for controlling the ventilator (1) and for setting the target oxygen concentration in the breathing gas depending on the ACTUAL oxygen saturation and/or the oxygen saturation target;
- a measuring device (30) for detecting the ACTUAL oxygen saturation of the living being (40) to be ventilated and/or the ACTUAL oxygen concentration, wherein the measuring device (30) preferably comprises a pulse oximeter for detecting the ACTUAL oxygen saturation; and
- a user interface for outputting information and/or inputting commands and/or data, wherein the user interface in particular comprises a numerical display;
wherein the regulation unit (20) is configured to carry out the following steps:
- detecting (130) the ACTUAL oxygen saturation of the living being (40) to be ventilated for a ventilation time;
- detecting (140) the ACTUAL oxygen concentration in the breathing gas for a ventilation time;
- defining (180) an oxygen saturation target;
- comparing (190) the ACTUAL oxygen saturation with the oxygen saturation target; and
- regulating (200) the oxygen concentration in the breathing gas to a target oxygen concentration depending on the comparison of the ACTUAL oxygen saturation with the oxygen saturation target, such that the ventilation takes place automatically on the basis of the detected ACTUAL oxygen saturation and detected ACTUAL oxygen concentration on the one hand and on the basis of the defined oxygen saturation target on the other hand, wherein the regulation of the oxygen concentration in the breathing gas comprises:
- regulating (200a) the ambient air flow in the air line (11); and/or
- regulating (200b) the oxygen flow in the oxygen line (12); and/or
- regulating (200c) the breathing gas in the breathing gas line (15);
- testing (150) the signal quality of the detected ACTUAL oxygen saturation;
**characterized in that**
the regulation (200) of the oxygen concentration in the breathing gas further comprises:
- raising (201) the oxygen concentration if the oxygen concentration of the breathing gas was not raised at least since a first waiting time; and/or
- lowering (202) the oxygen concentration if the oxygen concentration of the breathing gas was not lowered at least since a second waiting time;
and the definition (108) of the oxygen saturation target comprises:
- determining an average value of a set oxygen saturation target range; and
- defining the determined average value as the oxygen saturation target.

2. The ventilator (1) according to claim 1, wherein the regulation unit (20) is configured to carry out the following step:
- alerting (210) when
- the detected ACTUAL oxygen saturation is below the defined oxygen saturation target and/or
- the detected ACTUAL oxygen saturation is above the defined oxygen saturation target and/or
- the tested signal quality is insufficient, in particular if the signal quality is insufficient after a buffer time after being tested again;
and/or to carry out the following step:
- outputting at least one of the following items of information to a user interface of the ventilator (1):
- the progression of the ACTUAL oxygen concentration in the breathing gas,
- the length of time for which the oxygen concentration was substantially kept unchanged during the regulation,
- the length of time since a last change in the oxygen concentration,
- the ACTUAL oxygen saturation,
- the oxygen saturation target,
- the limit oxygen concentration;

3. The ventilator (1) according to any one of the preceding claims, wherein the raising (201) of the oxygen concentration comprises at least one of the following steps:
- raising the ACTUAL oxygen concentration to 100% if the detected ACTUAL oxygen saturation is 10% below the defined oxygen saturation target;
- raising the ACTUAL oxygen concentration by 15% if the detected ACTUAL oxygen saturation is 6% to 10% below the defined oxygen saturation target;
- raising the ACTUAL oxygen concentration by 10% if the detected ACTUAL oxygen saturation is 4% to 6% below the defined oxygen saturation target;
- raising the ACTUAL oxygen concentration by 5% if the detected ACTUAL oxygen saturation is 2% to 3% below the defined oxygen saturation target;
- raising the ACTUAL oxygen concentration by 1% if the detected ACTUAL oxygen saturation is 1% to 2% below the defined oxygen saturation target;
and/or wherein the lowering (202) of the oxygen concentration comprises at least one of the following steps:
- lowering the ACTUAL oxygen concentration to 10% if the detected ACTUAL oxygen saturation is 10% or 6% to 10% or 4% to 6% above the defined oxygen saturation target;
- lowering the ACTUAL oxygen concentration by 5% if the detected ACTUAL oxygen saturation is 2% to 3% above the defined oxygen saturation target;
- lowering the ACTUAL oxygen concentration by 1% if the detected ACTUAL oxygen saturation is 1% to 2% above the defined oxygen saturation target.

4. The ventilator (1) according to any one of the preceding claims, wherein the raising (201) of the oxygen concentration further takes place if
- the detected ACTUAL oxygen saturation is below the defined oxygen saturation target and/or
- a progression of the detected ACTUAL oxygen saturation decreases within a trend period;
and/or wherein the lowering (202) of the oxygen concentration further takes place if
- the detected ACTUAL oxygen saturation is above the defined oxygen saturation target and/or
- a progression of the detected ACTUAL oxygen saturation increases within a trend period;
and/or wherein the regulation (200) of the oxygen concentration in the breathing gas further comprises: maintaining (203) the oxygen concentration if the detected ACTUAL oxygen saturation substantially corresponds to the defined oxygen saturation target.

5. The ventilator (1) according to claim 4, wherein the regulation (200) of the oxygen concentration only takes place if the signal quality of the detected ACTUAL oxygen saturation in the form of a perfusion index is above a particular lower limit value, wherein said lower limit value is between 0.15% and 0.30%.

6. The ventilator (1) according to any one of the preceding claims, wherein the regulation (200) of the oxygen concentration comprises the step of waiting for a time period before the step of raising (201) or lowering (202) the oxygen concentration is performed, wherein the determined values of the ACTUAL oxygen saturation are not taken into account during the time period for regulating the oxygen concentration and/or a step of maintaining (203) the oxygen concentration is carried out within the time period if the detected ACTUAL oxygen saturation corresponds substantially to the defined oxygen saturation target, and wherein the time period is between 1 second and 20 seconds, preferably between 5 seconds and 10 seconds.

7. The ventilator (1) according to any one of the preceding claims, wherein the method (101) comprises a step of checking the breath volume, wherein the step of raising (201) and/or the step of lowering (202) the regulation (200) of the oxygen concentration only takes place if the breath volume is determined as being sufficiently large, wherein the breath volume is adapted by adapting at least a ventilation pressure, wherein the adaptation of the ventilation pressure takes place by raising the inspiratory airway pressure (IPAP) and/or lowering the expiratory airway pressure (EPAP).

8. The ventilator (1) according to claim 1, wherein the regulation unit (20) is configured to carry out the following steps of a method (100) for initializing regulation of the ventilator (1):
- setting (110) an oxygen saturation target range of a living being (40) to be ventilated;
- setting (120) a limit oxygen concentration in the breathing gas;
- detecting (130) the ACTUAL oxygen saturation of the living being (40) to be ventilated and/or detecting (140) the ACTUAL oxygen concentration in the breathing gas for an initialization time; and
- testing (150) a signal quality of the detected ACTUAL oxygen saturation; wherein the oxygen saturation target is within the set oxygen saturation target range.

9. The ventilator (1) according to claim 8, wherein the oxygen saturation target range to be set is between an oxygen saturation of the living being (40) to be ventilated of at least 85% and at most 99%, wherein oxygen saturation target ranges to be set are preferably in the region of from 85% to 88%, 86% to 89%, 87% to 90%, 88% to 91%, 89% to 92%, 90% to 93%, 91% to 94%, 92% to 95%, 93% to 96%, 94% to 97%, 95% to 98%, or 96% to 99%.

10. The ventilator (1) according to claim 8 or 9, wherein the setting (120) of the limit oxygen concentration in the breathing gas comprises:
- applying an oxygen safety factor to the detected ACTUAL oxygen concentration, wherein the oxygen safety factor preferably corresponds to 20%.

11. The ventilator (1) according to any one of claims 8 to 10, wherein the regulation unit (20) is configured to carry out at least one of the following steps:
- starting the ventilation by confirming the set limit oxygen concentration in the breathing gas if the signal quality is suitable;
- outputting an item of warning information if the signal quality is unsuitable.

## Revendications

1. Appareil respiratoire (1) pour la ventilation d'un être vivant (40), comprenant
- une unité de mélange (10) pour l'enrichissement d'un air ambiant avec un gaz d'oxygène jusqu'à une concentration cible d'oxygène et pour la fourniture de l'air ambiant enrichi jusqu'à la concentration cible d'oxygène comme gaz respiratoire ;
- une conduite d'air (11) qui est raccordée à l'unité de mélange (10) et est conçue pour amener l'air ambiant à l'unité de mélange (10), l'appareil respiratoire (1) comprenant une unité de dosage d'air (13) pour l'ajustement du flux d'air ;
- une conduite d'oxygène (12) qui est raccordée à l'unité de mélange (10) et est conçue pour amener le gaz d'oxygène à l'unité de mélange (10), l'appareil respiratoire (1) comprenant une unité de dosage d'oxygène (14) pour l'ajustement du flux de gaz d'oxygène ;
- une conduite de gaz respiratoire (15) qui est raccordée à l'unité de mélange (10) et est conçue pour évacuer le gaz respiratoire hors de l'unité de mélange (10), l'appareil respiratoire (1) comprenant de préférence une unité de dosage de gaz respiratoire (16) pour l'ajustement d'un flux de gaz respiratoire ;
- une unité de régulation (20) pour la commande de l'appareil respiratoire (1) et pour l'ajustement de la concentration cible d'oxygène dans le gaz respiratoire en fonction de la saturation d'oxygène réelle et/ou de la saturation d'oxygène cible ;
- un dispositif de mesure (30) pour la saisie de la saturation d'oxygène réelle de l'être vivant (40) devant être ventilé et/ou de la concentration d'oxygène réelle, le dispositif de mesure (30) pour la saisie de la saturation d'oxygène réelle comprenant de préférence un oxymètre de pouls ; et
- une interface utilisateur pour la sortie d'informations et/ou l'entrée de commandes et/ou de données, l'interface utilisateur comprenant en particulier un affichage numérique ;
l'unité de régulation (20) étant configurée pour effectuer les étapes suivantes :
- saisir (130) la saturation d'oxygène réelle de l'être vivant (40) devant être ventilé pour un temps de ventilation ;
- saisir (140) la concentration d'oxygène réelle dans le gaz respiratoire pour un temps de ventilation ;
- déterminer (180) une saturation d'oxygène cible ;
- comparer (190) la saturation d'oxygène réelle et la saturation d'oxygène cible ; et
- réguler (200) la concentration d'oxygène dans le gaz respiratoire jusqu'à une concentration d'oxygène cible en fonction de la comparaison entre la saturation d'oxygène réelle et la saturation d'oxygène cible, de telle sorte que la ventilation est effectuée automatiquement sur la base de la saturation d'oxygène réelle saisie et de la concentration d'oxygène réelle saisie, d'une part, et de la saturation d'oxygène cible déterminée, d'autre part, la régulation de la concentration d'oxygène dans le gaz respiratoire comprenant :
- réguler (200a) le flux d'air ambiant dans la conduite d'air (11) ; et/ou
- réguler (200b) le flux d'oxygène dans la conduite d'oxygène (12) ; et/ou
- réguler (200c) le gaz respiratoire dans la conduite de gaz respiratoire (15) ;
- contrôler (150) la qualité du signal de la saturation d'oxygène réelle saisie ;
**caractérisé en ce que**
la régulation (200) de la concentration d'oxygène dans le gaz respiratoire comprend en outre :
- augmenter (201) la concentration d'oxygène lorsque la concentration d'oxygène du gaz respiratoire n'a pas été augmentée au moins depuis un premier temps d'attente ; et/ou
- baisser (202) la concentration d'oxygène lorsque la concentration d'oxygène du gaz respiratoire n'a pas été baissée au moins depuis un deuxième temps d'attente ;
et **en ce que** la détermination (108) de la saturation d'oxygène cible comprend :
- déterminer une valeur moyenne d'un intervalle de saturation d'oxygène cible ajusté ; et
- définir la valeur moyenne spécifique comme saturation d'oxygène cible.

2. Appareil respiratoire (1) selon la revendication 1, l'unité de régulation (20) étant configurée pour effectuer l'étape suivante :
- émettre une alarme (210) lorsque
- la saturation d'oxygène réelle saisie est inférieure à la saturation d'oxygène cible définie et/ou
- la saturation d'oxygène réelle saisie est supérieure à la saturation d'oxygène cible définie et/ou
- la qualité du signal contrôlée est insuffisante, en particulier lorsque, après un nouveau contrôle, la qualité du signal est insuffisante après une marge de temps ;
et/ou pour effectuer l'étape suivante :
- sortir au moins l'une des informations suivantes sur une interface utilisateur de l'appareil respiratoire (1) :
- évolution de la concentration d'oxygène réelle dans le gaz respiratoire,
- durée pendant laquelle la concentration d'oxygène a été maintenue essentiellement inchangée pendant la régulation,
- durée depuis la dernière modification de la concentration d'oxygène,
- la saturation d'oxygène réelle,
- la saturation d'oxygène cible,
- la concentration d'oxygène limite ;
et/ou pour effectuer l'étape suivante :
- entrer au moins l'une des commandes suivantes sur l'interface utilisateur de l'appareil respiratoire (1) :
- interrompre la régulation de la concentration d'oxygène dans le gaz respiratoire, en particulier interrompre la régulation de la concentration d'oxygène pour une gazométrie sanguine ;
- activer et/ou désactiver la saisie de la saturation d'oxygène réelle.

3. Appareil respiratoire (1) selon l'une des revendications précédentes, l'augmentation (201) de la concentration d'oxygène comprenant au moins l'une des étapes suivantes :
- augmentation de la concentration d'oxygène réelle jusqu'à 100 % lorsque la saturation d'oxygène réelle saisie est inférieure de 10 % à la saturation d'oxygène cible définie ;
- augmentation de la concentration d'oxygène réelle de 15 % lorsque la saturation d'oxygène réelle saisie est inférieure de 6 % à 10 % à la saturation d'oxygène cible définie ;
- augmentation de la concentration d'oxygène réelle de 10 % lorsque la saturation d'oxygène réelle saisie est inférieure de 4 % à 6 % à la saturation d'oxygène cible définie ;
- augmentation de la concentration d'oxygène réelle de 5 % lorsque la saturation d'oxygène réelle saisie est inférieure de 2 % à 3 % à la saturation d'oxygène cible définie ;
- augmentation de la concentration d'oxygène réelle de 1 % lorsque la saturation d'oxygène réelle saisie est inférieure de 1 % à 2 % à la saturation d'oxygène cible définie ;
et/ou la baisse (202) de la concentration d'oxygène comprenant au moins l'une des étapes suivantes :
- baisse de la concentration d'oxygène réelle jusqu'à 10 % lorsque la saturation d'oxygène réelle saisie est supérieure de 10 % ou de 6 % à 10 % ou de 4 % à 6 % à la saturation d'oxygène cible définie ;
- baisse de la concentration d'oxygène réelle de 5 % lorsque la saturation d'oxygène réelle saisie est supérieure de 2 % à 3 % à la saturation d'oxygène cible définie ;
- baisse de la concentration d'oxygène réelle de 1 % lorsque la saturation d'oxygène réelle saisie est supérieure de 1 % à 2 % à la saturation d'oxygène cible définie.

4. Appareil respiratoire (1) selon l'une des revendications précédentes, l'augmentation (201) de la concentration d'oxygène étant en outre effectuée lorsque
- la saturation d'oxygène réelle saisie est inférieure à la saturation d'oxygène cible définie et/ou
- une évolution de la saturation d'oxygène réelle saisie diminue au cours d'une durée de tendance ;
et/ou la baisse (202) de la concentration d'oxygène étant effectuée lorsque
- la saturation d'oxygène réelle saisie est supérieure à la saturation d'oxygène cible définie et/ou
- une évolution de la saturation d'oxygène réelle saisie augmente au cours d'une durée de tendance ;
et/ou la régulation (200) de la concentration d'oxygène dans le gaz respiratoire comprenant en outre : Maintenir (203) la concentration d'oxygène lorsque la saturation d'oxygène réelle saisie correspond essentiellement à la saturation d'oxygène cible définie.

5. Appareil respiratoire (1) selon la revendication 4, la régulation (200) de la concentration d'oxygène n'étant effectuée que lorsque la qualité du signal de la saturation d'oxygène réelle saisie sous la forme d'un indice de perfusion est supérieure à une valeur limite inférieure déterminée, cette valeur limite inférieure se trouvant entre 0,15 % et 0,30 %.

6. Appareil respiratoire (1) selon l'une des revendications précédentes, la régulation (200) de la concentration d'oxygène comprenant l'étape d'attendre pendant un intervalle de temps avant que l'étape de l'augmentation (201) ou de la baisse (202) de la concentration d'oxygène soit effectuée, les valeurs déterminées de la saturation d'oxygène réelle n'étant pas prises en compte pour la régulation de la concentration d'oxygène pendant l'intervalle de temps et/ou une étape de maintien (203) de la concentration d'oxygène étant effectuée pendant l'intervalle de temps lorsque la saturation d'oxygène réelle saisie correspond essentiellement à la saturation d'oxygène cible définie, et l'intervalle de temps se trouvant entre 1 seconde et 20 secondes, de préférence entre 5 secondes et 10 secondes.

7. Appareil respiratoire (1) selon l'une des revendications précédentes, le procédé (101) comprenant une étape de vérification d'un volume d'inspiration, l'étape de l'augmentation (201) et/ou l'étape de la baisse (202) de la régulation (200) de la concentration d'oxygène étant effectuée uniquement lorsque le volume d'inspiration est déterminé comme étant suffisamment important, le volume d'inspiration étant ajusté par un ajustement au moins d'une pression de ventilation, l'ajustement de la pression de ventilation étant effectué par une augmentation de la pression inspiratoire des voies respiratoires (IPAP) et/ou par une baisse de la pression expiratoire des voies respiratoires (EPAP).

8. Appareil respiratoire (1) selon la revendication 1, l'unité de régulation (20) étant configurée pour effectuer les étapes suivantes d'un procédé (100) pour l'initialisation de la régulation de l'appareil respiratoire (1) :
- ajustement (110) d'un intervalle de saturation d'oxygène cible d'un être vivant (40) devant être ventilé ;
- ajustement (120) d'une concentration d'oxygène limite dans le gaz respiratoire ;
- saisir (130) la saturation d'oxygène réelle de l'être vivant (40) devant être ventilé et/ou saisir (140) la concentration d'oxygène réelle dans le gaz respiratoire pour un temps d'initialisation ; et
- contrôler (150) une qualité de signal de la saturation d'oxygène réelle saisie ; la saturation d'oxygène cible se trouvant dans l'intervalle de saturation d'oxygène cible ajusté.

9. Appareil respiratoire (1) selon la revendication 8, l'intervalle de saturation d'oxygène cible devant être ajusté se trouvant entre une saturation d'oxygène de l'être vivant (40) devant être ventilé d'au moins 85 % et au maximum de 99 %, les intervalles de saturation d'oxygène cible devant être ajustés se trouvant de préférence dans la plage de 85 % à 88 %, de 86 % à 89 %, de 87 % à 90 %, de 88 % à 91 %, de 89 % à 92 %, de 90 % à 93 %, de 91 % à 94 %, de 92 % à 95 %, de 93 % à 96 %, de 94 % à 97 %, de 95 % à 98 % ou de 96 % à 99 %.

10. Appareil respiratoire (1) selon la revendication 8 ou 9, l'ajustement (120) de la concentration limite d'oxygène dans le gaz respiratoire comprenant :
- appliquer un facteur de sécurité d'oxygène à la concentration d'oxygène réelle saisie, le facteur de sécurité d'oxygène étant de préférence de 20 %.

11. Appareil respiratoire (1) selon l'une des revendications 8 à 10, l'unité de régulation (20) étant configurée pour effectuer au moins l'une des étapes suivantes :
- commencer la ventilation par la confirmation de la concentration limite d'oxygène ajustée dans le gaz respiratoire lorsque la qualité du signal est adéquate ;
- sortir une information d'alerte lorsque la qualité du signal n'est pas adéquate.
